# EUROPEAN PATENT SPECIFICATION

(11) **EP 2 990 045 B1**
(45) Date of publication and mention of the grant of the patent: **16.11.2016**
(21) Application number: 14182253.6
(22) Date of filing: 26.08.2014
(51) Int. Cl.: A61K 35/745, A61P 1/00

(54) **Bifidobacterium animalis subsp. lactis for use in decreasing abdominal pain in a postmenopausal woman**
Bifidobacterium animalis subsp. lactis zur Verwendung bei der Verminderung von Unterleibsschmerzen bei einer Frau in der Postmenopause
Bifidobacterium animalis subsp. lactis destiné à être utilisé pour réduire la douleur abdominale chez une femme ménopausée

(43) Date of publication of application: 02.03.2016
(73) Proprietor: Chr. Hansen A/S, 2970 Hoersholm (DK)
(72) Inventor: Eskesen, Dorte, 2840 Holte (DK); Jespersen, Lillian, 2730 Herlev (DK); Morberg, Cathrine Melsaether, 2970 Hoersholm (DK); Michelsen, Birgit, 3450 Alleroed (DK)

(56) References cited:
- WO-A1-2010/008272
- GUYONNET D ET AL: "Effect of a fermented milk containing Bifidobacterium animalis DN-173 010 on the health-related quality of life and symptoms in irritable bowel syndrome in adults in primary care: a multicentre, randomized, double-blind, controlled trial", ALIMENTARY PHARMACOLOGY & THERAPEUTICS, BLACKWELL SCIENTIFIC PUBLICATIONS LTD., CAMBRIDGE, GB, vol. 26, no. 3, 1 August 2007 (2007-08-01) , pages 475-486, XP002479503, ISSN: 0269-2813
- SONDERGAARD BO ET AL: "Effects of probiotic fermented milk on symptoms and intestinal flora in patients with irritable bowel syndrome: a randomized, placebo-controlled trial.", SCANDINAVIAN JOURNAL OF GASTROENTEROLOGY JUN 2011, vol. 46, no. 6, June 2011 (2011-06), pages 663-672, XP009181577, ISSN: 1502-7708

## Description

### FIELD OF THE INVENTION

The present invention is based upon the findings of two large clinical studies to determine the efficacy of daily consumption of the probiotic strain *Bifidobacterium animalis* subsp. *lactis* deposited as DSM 15954 (BB-12^{®}), for 4 weeks on abdominal pain in healthy women with abdominal discomfort and a low defecation frequency.

### BACKGROUND OF THE INVENTION

Studies have investigated the effect of *Bifidobacterium animalis* subspecies *lactis* on abdominal pain in healthy subjects. However, none of the studies provide separate analyses of the effect in pre- and postmenopausal women.

### SUMMARY OF THE INVENTION

Episodes of abdominal pain or discomfort occur both in healthy people and in individuals suffering from irritable bowel syndrome, IBS, the difference being the higher frequency and greater severity of the symptoms in IBS patients (EFSA Journal 2011;9(4):1984)).

The present invention is based upon the results of two large clinical studies in healthy subjects with abdominal discomfort and a low defecation frequency. The results of these studies demonstrate an effect of *Bifidobacterium animalis* subsp. *lactis* compared to placebo on abdominal pain in postmenopausal women which is significantly higher than in premenopausal women.

In accordance herewith, the present invention relates to a probiotic product comprising *Bifidobacterium animalis* subsp. *lactis* for use in decreasing abdominal pain in a postmenopausal woman.

### DETAILED DISCLOSURE OF THE INVENTION

In this specification the following terms have the following meanings:
The administration of probiotics, defined by the World Health Organization as "live organisms which when administered in adequate amounts confer a benefit on the host" is a safe nutritional intervention in the general healthy population. Probiotic strains are often used in yogurt and other food items (e.g., beverages, cereals and chocolate candy bars).

Probiotics have been investigated for their ability to modulate immune function, and positively impact clinical outcomes related to atopic allergies, respiratory allergies, respiratory tract infections, gastrointestinal conditions, periodontal infections, and female urogenital conditions.

By the term "a probiotic product" is meant any product which comprises a probiotic bacterium.

A probiotic product for use in accordance with the invention may be administered in the form of a food product or a dietary supplement. The *Bifidobacterium animalis* subsp. *lactis* may, for example, be incorporated in a dairy product, such as milk, and in particular a fermented dairy product, optionally in combination with other lactic acid bacteria, for example with yogurt ferments, or in other food products such as a snack bar, or beverages such as juice.

The probiotic product comprising *Bifidobacterium animalis* subsp. *lactis* can also be provided as a dietary supplement in the form of a powder, tablet, such as a lozenge or effervescent tablet, pastille, capsule, chewing gum, in individual sachets or as a component of a more general composition such as oil drops, an emulsion or a paste, or in any other suitable carrier determined by those of skill in the art to be an effective carrier for live microorganisms.

In a preferred embodiment, the probiotic product for use according to the invention comprises *Bifidobacterium animalis* subsp. *lactis* deposited as DSM 15954 (BB-12^{®}). *Bifidobacterium animalis* subsp *lactis* is commercially available from Chr. Hansen A/S, Hoersholm, Denmark and from Deutsche Sammlung von Mikroorganismen und Zellkulturen GmbH (DSMZ).

It is contemplated that the findings are generalizable to other *Bifidobacterium animalis* subspecies *lactis* strains, such as strains derived from, mutants and variants of the tested strain. Examples of such other strains of *Bifidobacterium animalis* subspecies *lactis* are DSM 10140, which is the B. *animalis* subsp. *lactis* type strain, HN019 also known as DR10™ and deposited with accession number DSM17280, Bi-04 also known as DGCC2908 and RB 4825, Bi-07 deposited as ATCC PTA-4802, ATCC 27536, NCC 2818, CNCM I-3446, VTT E-012010, and DN-173-010 deposited at CNCM with accession number I-2494.

Preferably, in order to obtain an optimal effect, the probiotic product should be administered daily for at least one week, and advantageously for a longer period such as at least 2 weeks, at least 4 weeks as in the present study, at least 6 weeks, at least 9 weeks, and preferably 12 weeks, in an amount corresponding to at least 10⁶ CFU, such as at least 10⁷ CFU, preferably at least 10⁸ CFU, generally between 10⁹ CFU and 10¹² CFU of *Bifidobacterium animalis* subsp. *lactis.*

The findings reported in the present patent application are based upon a subgroup of women having the ages of 18 to 70 years but are considered applicable also to women of the age above 70 years.

In the present studies the probiotic product comprises *Bifidobacterium animalis* subsp. *lactis* as the active ingredient. *Bifidobacterium animalis* subsp. *lactis* may be used as the only active ingredient. Alternatively, the probiotic product as described herein may comprise further compounds of interest such as other bacterial strains, vitamins, prebiotics, fibers or other compounds which may have a beneficial health effect.

The other bacterium may be selected from the group consisting of *Bifidobacterium lactis, Lactobacillus rhamnosus, Lactococcus lactis subsp. lactis, Lactococcus lactis* subsp. *cremoris, Leuconostoc lactis, Leuconostoc mesenteroides* subsp. *cremoris, Pediococcus pentosaceus, Lactococcus lactis* subsp. *lactis biovar. diacetylactis, Lactobacillus casei* subsp. *casei, Streptococcus thermophilus, Bifidobacterium longum, Lactobacillus lactis, Lactobacillus helveticus, Lactobacillus fermentum, Lactobacillus salivarius, Lactobacillus delbrueckii* subsp. *bulgaricus* and *Lactobacillus acidophilus.*

Thus, the composition may further comprise one or more strain(s) of a lactic acid bacterium selected from the group comprising *Lactobacillus acidophilus* deposited as DSM 13241 (LA-5^{®}), *Lactobacillus rhamnosus* deposited as ATCC 53103 (LGG^{®}), *Lactobacillus rhamnosus* deposited as ATCC 55826 (GR-1^{®}), *Lactobacillus reuteri* deposited as ATCC 55845 (RC-14^{®}), *Lactobacillus paracasei* subsp. *paracasei* deposited as ATCC 55544 (L.casei 431^{®}), *Lactobacillus paracasei* deposited as LMG-17806 (F19^{®}), *Streptococcus thermophilus* deposited as DSM 15957 (THE-4^{®}), *Lactobacillus fermentum* deposited as NM02/31074 (PCC^{®}), *Lactobacillus paracasei* subsp. *paracasei* deposited as CCTCC M204012 (LP-33^{®}).

### EXAMPLES

Two clinical studies have been conducted to investigate the effect of *Bifidobacterium animalis* subsp. *lactis* on abdominal pain in subjects with a low defecation frequency and with abdominal discomfort. One study tested the effect of *Bifidobacterium animalis* subsp. *lactis* in an acidified milk drink. The placebo product was a similar milk drink but without *Bifidobacterium animalis* subsp. *lactis.* In the other study, the test product was a capsule containing *Bifidobacterium animalis* subsp. *lactis.* The placebo product was a similar capsule but without the probiotic strain. The design of the two studies was identical. The results reported here are results from a meta-analysis of the two studies.

Subjects eligible for participation in the studies were healthy men or women of ≥18 to ≤70 years of age with a body mass index (BMI) between 19-35 kg/m² (both inclusive). In order to fulfill the inclusion criteria the subject should have experienced general abdominal discom-fort/complaints weekly during the month prior to study entry and have a stool frequency between on average 2-4 days/week with any number of defecations on these days.

Subjects who had a history or diagnosis of GI disease (e.g. gastric or duodenal ulcers, irritable bowel disease, colon cancer) or IBS or history of complicated GI surgery that could have an effect on GI tract function or a change of dietary habits within 4 weeks prior to the screening visit, e.g. start of fiber-enriched diet were not eligible for the studies.

The first visit was followed by a 2-week run-in phase. A two week baseline period is believed to be sufficient to record baseline data in subjects with minimal severity requirements before randomization. Further, the run-in period ensured a two week wash-out of any pre-study probiotics. After this run-in phase the subject was randomly assigned to receive either the test or the reference product, followed by a 4-week intervention phase during which the subjects consumed either the test or the reference product.

The subjects were not allowed to consume any fermented dairy products or probiotic products other than the study products supplied to them by the study personnel.

Compliance was based on subject's daily documentation of consumption of study product in the diary and number of returned capsules. Subjects who consumed >70% of the products, i.e. at least 5 of 7 days on a weekly basis, were considered compliant.

From 2 weeks prior to the screening visit (Visit 1) until final examination (Visit 4) all medications including OTC products, large doses of vitamins and minerals, and food or herbal supplements that could have an effect on GI well-being, gut microbiota, gut regularity or gut symptoms, in particular laxatives were prohibited, unless the subject had taken them at a stable dose from 4 weeks prior to Visit 1 and was going to take them throughout the study. Antibiotics and antimicrobial medications were prohibited from 4 weeks prior to the screening visit (Visit 1) until the end of the study (Visit 4). Hormonal contraceptives and the occasional use of paracetamol were allowed.

### Results

The results are provided in the following table showing the results in the post-menopausal subgroup, pooled data from ITT population.

**Table 1 Abdominal pain sum score**

| | Abdominal pain | *Bifidobacterium animalis* subsp. *lactis* 1 billion CFU | Placebo |
|---|---|---|---|
| Female (post), N=219 | Baseline | N=93 | N=126 |
| | Mean (SD) | 10.3 (5.4) | 10.6 (5.7) |
| | Median | 10 | 11 |
| | Week 4 | | |
| | Mean (SD) | 4.8 (5.4) | 6.5 (5.4) |
| | Median | 3 | 6 |
| | | | |
| | p-value¹ | 0.0090 | |

| | | | |
|---|---|---|---|
| ITT intention-to-treat; CFU = colony forming units; N number of subjects; SD standard deviation; ¹ p-values from analysis of variance of ranked data from week 4 using baseline value and study as covariates. | | | |

As can be seen from the table, *Bifidobacterium animalis* subsp. *lactis* significantly reduced abdominal pain.

## Claims

1. A probiotic product comprising *Bifidobacterium animalis* subsp. *lactis* for use in reducing abdominal pain in a postmenopausal woman.

2. A product for use according to claim 1, wherein the *Bifidobacterium animalis* subsp. *lactis* is deposited as DSM 15954.

3. A product for use according to claim 1 or 2, wherein the *Bifidobacterium animalis* subsp. *lactis* is the only active ingredient.

4. A product for use according to any one of claims 1 to 3 wherein the *Bifidobacterium animalis* subsp. *lactis* is administered in a capsule.

5. A probiotic product for use according to any one of claims 1 to 4, wherein the *Bifidobacterium animalis* subsp. *lactis* is administered in an amount corresponding to at least 10⁹ CFU.

6. A product for use according to any one of claims 1 to 5 wherein the product is administered for at least 2 weeks.

## Patentansprüche

1. Probiotisches Produkt, das ein *Bifidobacterium animalis* subsp. *lactis* enthält, zur Verwendung bei der Verminderung von Unterleibsschmerzen bei einer Frau in der Postmenopause.

2. Produkt zur Verwendung nach Anspruch 1, wobei das *Bifidobacterium animalissubsp. lactis* als DSM 15954 hinterlegt ist.

3. Produkt zur Verwendung nach Anspruch 1 oder 2, wobei das *Bifidobacterium animalis* subsp. *lactis* der einzige Wirkstoff ist.

4. Produkt zur Verwendung nach einem der Ansprüche 1 bis 3, wobei das *Bifidobacterium animalis* subsp. *lactis* in einer Kapsel verabreicht wird.

5. Probiotisches Produkt zur Verwendung nach einem der Ansprüche 1 bis 4, wobei das *Bifidobacterium animalis* subsp. *lactis* in einer Menge, die mindestens 10⁹ CFU entspricht, verabreicht wird.

6. Produkt zur Verwendung nach einem der Ansprüche 1 bis 5, wobei das Produkt für mindestens 2 Wochen verabreicht wird.

## Revendications

1. Produit probiotique comprenant *Bifidobacterium animalis* subsp. *lactis* pour l'utilisation pour la réduction des douleurs abdominales chez la femme en postménopause.

2. Produit pour l'utilisation selon la revendication 1, où le *Bifidobacterium animalis* subsp. *lactis* est déposé sous le numéro DSM 15954.

3. Produit pour l'utilisation selon la revendication 1 ou 2, où le *Bifidobacterium animalis* subsp. *lactis* est la seule substance active.

4. Produit pour l'utilisation selon l'une quelconque des revendications 1 à 3, où le *Bifidobacterium animalis* subsp. *lactis* est administré en capsule.

5. Produit probiotique pour l'utilisation selon l'une quelconque des revendications 1 à 4, où le *Bifidobacterium animalis* subsp. *lactis* est administré en une quantité qui correspond à au moins 10⁹ UFC.

6. Produit pour l'utilisation selon l'une quelconque des revendications 1 à 5, où le produit est administré pendant au moins 2 semaines.
